# EUROPEAN PATENT SPECIFICATION

(11) **EP 3 357 522 B1**
(45) Date of publication and mention of the grant of the patent: **16.10.2019**
(21) Application number: 17206101.2
(22) Date of filing: 17.03.2014
(51) Int. Cl.: A61L 31/16, A61L 15/22, A61L 15/44, A61L 29/08, A61L 29/16, A61L 31/10, A61P 31/04

(54) **COATED MEDICAL ARTICLE FOR DRUG RELEASE**
BESCHICHTETER MEDIZINISCHER ARTIKEL ZUR WIRKSTOFFFREISETZUNG
ARTICLE MEDICAL AYANT UN REVÊTEMENT POUR LIBÉRATION DE MÉDICAMENTS

(30) Priority: 15.03.2013 US 201361799859 P
(43) Date of publication of application: 08.08.2018
(62) Divisional of application: 14764271.4
(73) Proprietor: Interface Biologics Inc., Toronto, Ontario M5G 1L7 (CA)
(72) Inventor: SANTERRE, Joseph Paul, Toronto, ON, M4Y 1G3 (CA); ESFAND, Roseita, Mississauga, Ontario L5J 0A3 (CA)
(74) Representative: Steinecke, Peter

(56) References cited:
- WO-A1-03/040104
- CA-A1- 2 467 321
- CA-A1- 2 571 320
- US-A1- 2010 062 974

## Description

### FIELD OF THE INVENTION

This invention relates to compounds that include biologically active agents that can be used for effective drug release, e.g., as coatings for medical devices.

### BACKGROUND OF THE INVENTION

The appropriate biological response to the surface of a device is crucial for biocompatibility. The coating of a medical device using, e.g., organic compositions, can also serve as a repository for delivery of a biologically active agent. A coating that is used to control release of the drug must be free of impurities that trigger adverse biological responses (i.e., biologically inert), must produce the desired release profile, and must not adversely affect the mechanical properties required of the medical device. Further, when the active agent is a pharmaceutical drug, it is often desirable to release the drug locally from the medical device over an extended period of time.

Systems for kinetically controlled direct drug delivery can employ a polymer that includes a biologically active agent. For example, when the agent is part of the polymer backbone, it may be released as the polymer enzymatically degrades or disintegrates in the body. Drug release by such polymers, however, may be complicated by release of other organic entities, including various biologically active species resulting from incomplete hydrolysis. Alternatively, biologically active agents can be simply mixed with a polymer platform in a suitable solvent system. The biologically active agent is then released by particle dissolution or diffusion (when the non-bioerodable matrices are used) or during polymer breakdown (when a biodegradable polymer is used). In these systems the polymer coating will become part of the device design. Mixing lowers the entropy and this can result in phase separation throughout the bulk polymer, compromising the physical/mechanical properties of the polymeric coating. In addition the presence, stability, and uniform distribution of the drug throughout the polymeric coating can compromise the device performance (e.g., orthopedic devices). WO 03/040104 A1 discloses dimeric pharmaceutical compounds comprising two pharmaceutically active moieties (*e*.*g*. anti-inflammatory agents) linked through a linker formed, *e*.*g*., from a diamine or diol but not their use as coating on an implantable device. Each of documents CA 2571 320 and CA 2 467 321 discloses a compound of formula (I) which includes two biologically active agents which maybe the same or different and which are linked via an oligomeric segment LinkA, of 60 to 2000 Da. The oligomer linker group is, for example, triethylene glycol. CA 2 571 320 mentions CIPRO-TEGNORF, and CIPRO-TEG-CIPRO and CA 2 467 321 mentions CIPRO-TEG-CIPRO and CIPRO-TEG-NORF. US 2010/062974 discloses bioactive monomers comprising a membrane active biocide and a second antimicrobial agent, *e*.*g*., floroquinolone antibiotic such as norfloxacin with a coupling segment having a molecular weight of less than 2000 Da synthesized from dials or diamines. However the biomonomers disclosed in any of these documents are not used as such but as intermediates in synthesis of polymers which are either used alone or admixed with suitable amounts of base polymers in the fabrication of implantable articles.

In view of the potential drawbacks to current strategies for drug release by, e.g., coated devices, there exists a need for drug delivery platforms which provide for delivery of biologically active agents with a defined profile of release. The present invention addresses these problems and offers advantages over the current technology.

### SUMMARY OF THE INVENTION

The invention relates to the embodiments characterized in the claims and features an article that includes a coated surface, where said coated surface includes a compound having a structure according to formula (I):

Bio¹-Link¹-(Bio²-R¹)ₘ (I),

or a pharmaceutically acceptable salt thereof, where
Bio¹ and each Bio² are formed from a biologically active agent;
m is 1, 2, 3, 4, or 5;
each Bio² includes a covalent bond to Link¹;
R¹ is absent;
Link¹ is an oligomeric organic, organosilicon, or organosulfone segment having a molecular weight between 60 and 2000 Daltons; wherein said article is an implantable or a percutaneous medical device; and wherein said biologically active agent is an anti-inflammatory agent.

In some embodiments, the compound has a structure according to formula (I-A),

Bio¹-Link¹-Bio²-R¹ (I-A),

or a pharmaceutically acceptable salt thereof, where
Bio¹ and Bio² are formed from a biologically active agent;
R¹ is absent; and
Link¹ is an oligomeric organic, organosilicon, or organosulfone segment having a molecular weight between 60 and 2000 Daltons.

In certain embodiments, Bio¹ and Bio² are formed from biologically active agents that have the same structure.

In still other embodiments, Bio¹ and Bio² are formed from biologically active agents that have different structures.

In further embodiments, each Bio¹ and Bio², when present, has a molecular weight ranging from 100 to 1000, from 200 to 1000, from 200 to 900, from 200 to 800, from 200 to 700, from 200 to 600, from 200 to 500, or from 200 to 400 Daltons.

In certain embodiments, Link¹ has a molecular weight between 60 and 700 Daltons.

In other embodiments, Link¹ is formed from a diol, a diamine, or an α,ω-aminoalcohol.

In particular embodiments, Link¹ is formed from a diol.

In still other embodiments, Link¹ is formed from a polyethylene oxide having terminal amino or hydroxyl groups, and where Link¹ includes 1-3, 1-5, 1-10, or 1-20 ethylene oxide repeating units.

In some embodiments, Link¹ is formed from a compound selected from the group consisting of: ethylene glycol; butane diol; hexane diol; hexamethylene diol; 1,5-pentanediol; 2,2-dimethyl-I,3 propanediol; 1,4-cyclohexane diol; 1,4-cyclohexanedimethanol; tri(ethylene glycol); poly(ethylene glycol), where the molecular weight is between 100 and 2000 Daltons; poly(ethylene oxide) diamine, where the molecular weight is between 100 and 2000 Daltons; lysine esters; silicone diols; silicone diamines; polyether diols; polyether diamines; carbonate diols; carbonate diamines; dihydroxy vinyl derivatives; dihydroxydiphenylsulfone; ethylene diamine; hexamethylene diamine 1,2-diamino-2-methylpropane; 3,3-diamino-n-methyldipropylamine; 1,4-diaminobutane; 1,7-diaminoheptane; and 1,8-diaminooctane.

In particular embodiments, Link¹ is formed from tri(ethylene glycol).

In still other embodiments, Link¹ is formed from a dicarboxylic compound or a diisocyanate.

In particular embodiments, the coating includes a second compound having a structure according to formula (I) or formula (I-A), where each Bio¹, Link¹, and Bio² is as defined in any embodiment, or a combination of embodiments, described herein.

In still other embodiments, the coating is substantially free of any biologically active agent used to form Bio¹ and/or Bio² where the biologically active agent is not included in a compound according to formula (I) or formula (I-A).

In further embodiments, the coating further includes free biologically active agent, where the mole ratio of the compound according to formula (I) to the free biologically active agent is from 0.1:1 to 1:0.1.

In certain embodiments, compound according to formula (I) or formula (I-A) has reduced biological activity compared to the biologically active agent used to form Bio¹ and/or Bio².

In still other embodiments, the compound according to formula (I) or formula (I-A) has 0%-20% of the biological activity of the biologically active agent used to form Bio¹ and/or Bio².

In some embodiments, the coating includes a pharmaceutically acceptable salt of the compound according to formula (I) or formula (I-A).

In further embodiments, the pharmaceutically acceptable salt is the trifluoroacetate or the hydrochloride salt.

In certain embodiments, the article is a filter, film, fiber, sheet, or an implantable medical device.

In particular embodiments, the implantable device is selected from the group consisting of: prostheses pacemakers, electrical leads, defibrillators, artificial hearts, ventricular assist devices, anatomical reconstruction prostheses, artificial heart valves, heart valve stents, pericardial patches, surgical patches, coronary stents, vascular grafts, vascular and structural stents, vascular or cardiovascular shunts, biological conduits, pledges, sutures, annuloplasty rings, stents, staples, valved grafts, dermal grafts for wound healing, orthopedic spinal implants, orthopedic devices, ophthalmic implants, intrauterine devices, stents, maxial facial reconstruction plating, dental implants, intraocular lenses, clips, sternal wires, bone, skin, ligaments, sutures, hernia mesh, tendons, and combinations thereof

In other embodiments, the article is a percutaneous device selected from: catheters, cannulas, drainage tubes, and surgical instruments, or the article is a cutaneous device selected from burn dressings, wound dressings and dental hardware.

In some embodiments, the surgical instrument is selected from: forceps, retractors, needles, gloves, and catheter cuffs.

In other embodiments, the article is a catheter cuff.

In still other embodiments, the coating has a thickness between 0.5 to 120 µM.

In some embodiments, the article includes a fibrous polymer matrix that includes one or more compounds according to formula (I) and/or formula (I-A).

In particular embodiments, the article includes an admixture that includes a two or more compounds according to formula (I) and/or formula (I-A).

In certain embodiments, polymer matrix is formed from a biodegradable polymer.

In other embodiments, polymer is polylactic acid or polycaprolactone.

In some embodiments, polymer matrix is formed from a nonbiodegradable polymer.

In still other embodiments, the polymer is poly(ethylene terephthalate).

In certain embodiments, article is a catheter cuff.

In still other embodiments, the catheter cuff is a vascular access catheter cuff.

In further embodiments, the article is an orthopedic device.

In still other embodiments, orthopedic device is a wire, pin, rod, nail, screw, disk, plate, bracket, or splint.

In some embodiments, the ophthalmic implant is a punctal plug.

In particular embodiments, the article contains two or more compounds having a structure according to formula (I). In certain embodiments, the article contains two or more compounds having a structure according to formula (I-A). In other embodiments, the article contains one or more compounds having a structure according to formula (I) and one or more compounds having a structure according to formula (I-A).

The invention features a method for coating a surface, where the composition includes:
(a) a compound having a structure according to formula (I),

   Bio¹-Link¹-(Bio²-R¹)ₘ (I),

   or a pharmaceutically acceptable salt thereof, where
   Bio¹ is formed from a biologically active agent;
   m is 1, 2, 3, 4, or 5;
   each Bio² is independently formed from a biologically active agent, and where each Bio² includes a covalent bond to Link¹;
   R¹ is absent;
   Link¹ is an oligomeric organic, organosilicon, or organosulfone segment having a molecular weight between 60 and 2000 Daltons
      and
(b) a suitable medium in which the compound of (a) is soluble; and
   where said composition is substantially free of any biologically active agent used to form Bio¹ and/or Bio² where the biologically active agent is not included in a compound according to formula (I).

In some embodiments, the compound has a structure according to formula (I-A),

Bio¹-Link¹-Bio²-R¹ (I-A),

or a pharmaceutically acceptable salt thereof, where
Bio¹ is formed from a biologically active agent;
Bio² is formed from a biologically active agent;
R¹ is absent; and
Link¹ is an oligomeric organic, organosilicon, or organosulfone segment having a molecular weight between 60 and 2000 Daltons.

In particular embodiments, the compound has a structure according to any embodiment described herein for a compound according to formula (I) or formula (I-A), or combination of embodiments thereof.

In further embodiments, the component of (b) is an organic solvent or aqueous solvent.

In certain embodiments, the polar organic solvent is tetrahydrofuran, *N*,*N*-dimethylformamide, diethylamine, chloroform, methyl t-butyl ether, toluene, benzene, ether, p-xylene, carbon disulfide, carbon tetrachloride, cyclohexane, pentane, hexane, heptane, dioxane, ethylacetate, dimethoxyethane, ethyl benzoate, anisol, chlorobenzene, pyridine, acetone, dimethylsulfoxide, acetonitrile, ethanol, n-propanol, toluene, methanol, water, or benzyl alcohol.

In still other embodiments, the concentration of (a) is between 0.05-150 mg/mL.

In certain embodiments of any aspect of the invention, the article contains two or more compounds having a structure according to formula (I) and/or formula (I-A). In other embodiments, Bio¹ of the compound of formula (I) or (IA) is ciprofloxacin. In yet other embodiments, Bio¹ of the compound of formula (I) or (IA) is hydrocortisone.

In certain embodiment of any aspect of the invention, the molecular weight is a theoretical molecular weight.

By the term "oligomeric segment" is meant a relatively short length of a repeating unit or units, generally less than about 50 monomeric units and molecular weights less than 10,000 but preferably <5000. Oligomeric segments can be selected from the group consisting of polyurethane, polyurea, polyamides, polyalkylene oxide, polycarbonate, polyester, polylactone, polysilicone, polyethersulfone, polyolefin, polyvinyl, polypeptide, polysaccharide; and ether and amine linked segments thereof, or other multifunctional compounds as described herein. The linking segments (e.g., the Link¹ segments) described herein can include oligomeric segments.

Typically, Link (e.g., Link¹) molecules can have molecular weights ranging from 60 to 2000 and preferably 60-700, and have difunctionality to permit coupling of two oligo units. Preferably the Link molecules are synthesized from diamines, diisocyanates, disulfonic acids, dicarboxylic acids, diacid chlorides and dialdehydes. Terminal hydroxyls, amines or carboxylic acids on the oligo molecules can react with diamines to form oligo-amides; react with diisocyanates to form oligo-urethanes, oligo-ureas, oligo- amides; react with disulfonic acids to form oligo-sulfonates, oligo-sulfonamides; react with dicarboxylic acids to form oligo-esters, oligo-amides; react with diacid chlorides to form oligo-esters, oligo-amides; and react with dialdehydes to form oligo-acetal, oligoimines.

The terms "pharmaceutically active agent" and "biologically active agent", or precursor thereof, refer to a molecule that can be coupled to a Link segment via hydrolysable covalent bonding. Hydrolysable covalent bonds are those that can undergo spontaneous or catalyzed (e.g., enzyme-catalyzed) hydrolytic cleavage under physiological conditions (e.g., mammalian physiological conditions). Non-limiting examples of functional groups containing hydrolysable covalent bonds include: esters, thioesters, amides, thioamides, sulfonamides, sulfinamides, acid anhydrides, imides, imines, phosphate esters, and phosphonate esters. Accordingly, each biologically active agent used to form [Bio¹] and [Bio²], includes at least one group selected independently from the group consisting of carbonyl group, amine, phosphonate, phosphate, sulfonate, sulfinate, and a combinations thereof. Thus, the compounds of the invention, when implanted *in vivo* as part of a coating, undergo hydrolysis of one or more of the groups containing hydrolysable covalent bonds, thereby releasing defined degradation products consisting of biological, pharmaceutical, and/or biocompatible components. The molecule must have some specific and intended pharmaceutical or biological action. Typically the [Bio] unit has a molecular weight ranging from 40 to 2000 for pharmaceuticals but may be higher for biopharmaceuticals depending on the structure of the molecule. The Bio unit is an anti- inflammatory agent.

The term "pharmaceutically acceptable salt" as used herein, represents those salts which are, within the scope of sound medical judgment, suitable for use in contact with the tissues of humans and animals without undue toxicity, irritation, allergic response and the like and are commensurate with a reasonable benefit/risk ratio. Pharmaceutically acceptable salts are well known in the art. For example, S. M. Berge et al. describe pharmaceutically acceptable salts in detail in *J. Pharm. Sci.* 66:1-19, 1977. The salts can be prepared in situ during the final isolation and purification of the compounds of the invention or separately by reacting the free base group with a suitable organic acid. Representative acid addition salts include acetate, adipate, alginate, ascorbate, aspartate, benzenesulfonate, benzoate, bisulfate, borate, butyrate, camphorate, camphersulfonate, carbonate, chloride, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like.

The term "theoretical molecular weight" in this specification is the term given to the absolute molecular weight that would result from the reaction of the reagents utilized to synthesize any given bioactive polymers. As is well known in the art, the actual measurement of the absolute molecular weight is complicated by physical limitations in the molecular weight analysis of polymers using gel permeation chromatography methods. Hence, a polystyrene equivalent molecular weight is reported for gel permeation chromatography measurements. Since many biologically active compounds absorb light in the UV region, the gel permeation chromatography technique also provides a method to detect the distribution of biologically active compound coupled within polymer chains.

### BRIEF DESCRIPTION OF THE DRAWINGS

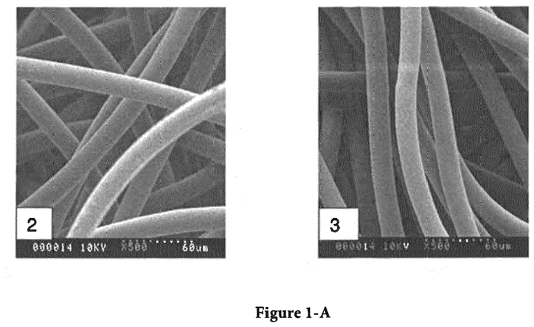

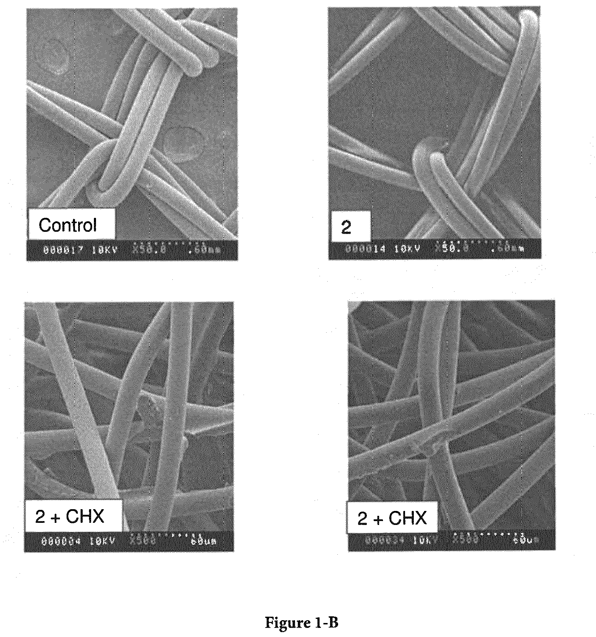

**Figures 1-A** **and** **1-B** show the SEM analysis of coated Dacron meshes and Hernia meshes coated with Compound 2 and Compound 3 in DMF, which showed a smooth coating with limited webbing. Dacron mesh coated with compound 2 and Chlorohexidine shows a smooth and uniform coating. Dacron meshes coated with Compound 2 or Compound 3 are shown in Figure 1-A. Hernia meshes (control) and those coated with Compound 2 or Compound 2 plus chlorhexidine are shown in Figure 1-B.

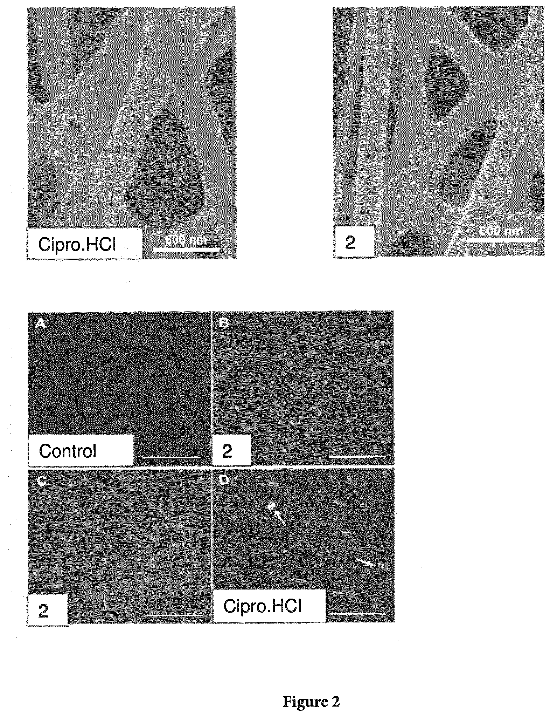

**Figure 2** shows the SEM and Confocal light microscopy images showing ciprofloxacin.HCl vs compound 2 distribution in scaffold fibers. Electro-spun material (polyurethane with Ciprofloxacin or compound 2) shows smooth and uniform coating with compound 2 vs the drug alone (electro-spun). SEM (Top images of Cipro.HCl (left) and Compound 2 in polymer admixture (right)), and Confocal light microscopy images (bottom panel) (A) Control Fiber, (B) Compound 2 and polymer admixture fiber, (C) Compound 2 and polymer admixture fiber and (D) Ciprofloxacin HCl and polymer admixture fiber. Aggregated drug is seen as non-fiber clumps (white arrows) in the polymer fibers containing drug alone. Scale bars = 50 µm.

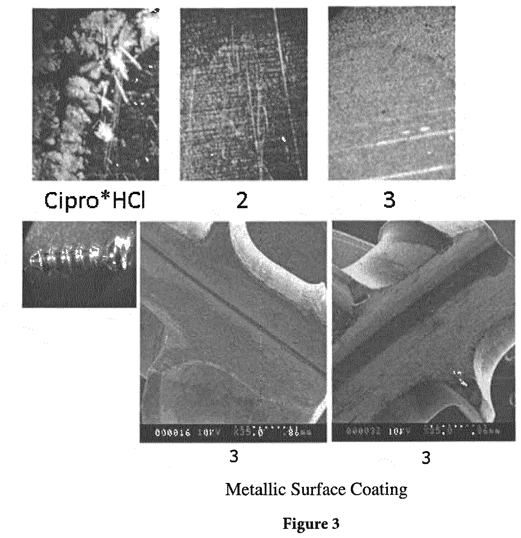

**Figure 3** shows stainless steel coupons and orthopedic screws that were dipped once for thirty seconds in either a 10 mg/mL solution of Compound 2 in organic solvent, Compound 3 in organic solvent, or ciprofloxacin hydrochloride in organic solvent, or DMF (control). Coupons with ciprofloxacin hydrochloride had a white uneven coating, while those coated with Compounds 2 and 3 were clear.

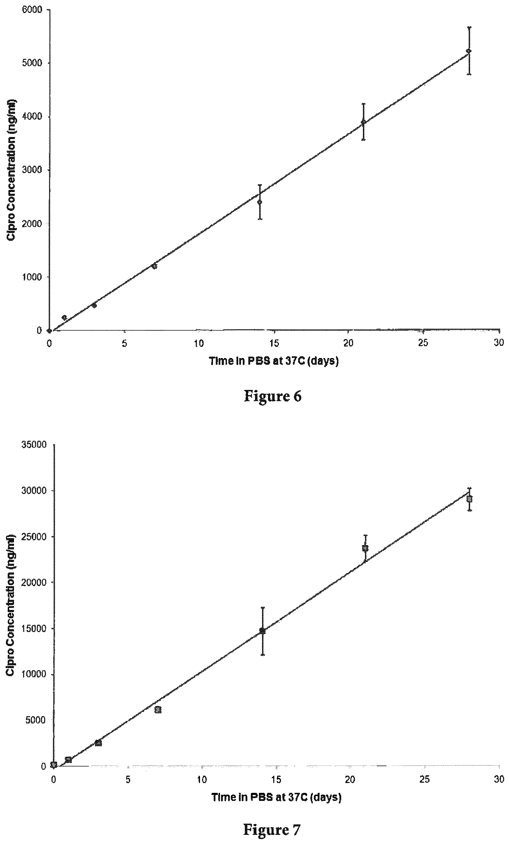

**Figure 6** relates to drug release from Compound 2 in PBS at 37 °C. After 28 days, ∼8% total drug was released from Compound 2, demonstrating slow and sustained release under these conditions.
**Figure 7** relates to drug release from Compound 3 in PBS at 37 °C. A linear increase in the drug concentration was observed with time out to at least 28 days.

### DETAILED DESCRIPTION OF THE INVENTION

This invention relates to compounds as characterized in the claims that include biologically active agents that can be used for effective drug release as coatings for medical devices. The biologically active agents include biologically active agents linked via oligomeric segments. The advantages of the invention include improved thermodynamic compatibility of drugs with processing agents, thereby providing: (i) the ability to form uniform coatings on polymeric and metallic surfaces without the complications of phase separation, and drug crystallization, When combined with other coating materials, such as base polymers; (ii) uniform distribution of drugs throughout the coatings when the compounds are used in admixture with polymers (e.g., base polymers) to form films, fibers, and extruded articles; (iii) localization of drugs at therapeutic concentrations; (iv) stability of drugs under processing and storage conditions; and (v) formulation in a stable liquid phase which can be used for further processing. An article of the invention may include a coated surface containing one or more (e.g., two or more) compounds of formula (I) or one or more (e.g., two or more) compounds of formula (I-A). Alternatively, an article of the invention may include a coated surface containing one or more (e.g., two or more) compound of formula (I) and one or more (e.g., two or more) compound of formula (I-A).

### Oligomeric Segments

The compounds described herein include a LINK¹ moiety, which is an oligomeric segment. By "oligomeric segment" or "Oligo" is meant a relatively short length of a repeating unit or units, generally fewer than about 50 monomeric units and molecular weights between 60 and 2000 Daltons. The LINK¹ moiety has multi-functionality, but preferably di-functionality, to permit covalent bond formation to, e.g., a biologically active agent such as Bio¹ and Bio². The coupling segments can be synthesized from the groups of precursor monomers selected from diols, diamines and/or a compounds containing both amine and hydroxyl groups. Precursors that can be incorporated into coupling segments include, without limitation, ethylene glycol, butane diol, hexane diol, hexamethylene diol, 1,5-pentanediol, 2,2-dimethyl-1,3 propanediol, 1 ,4-cyclohexane diol, I ,4-cyclohexanedimethanol, tri(ethylene glycol), poly(ethylene glycol), poly(ethylene oxide) diamine, lysine esters, silicone diols and diamines, polyether diols and diamines, carbonate diols and diamines, dihydroxy vinyl derivatives, dihydroxy diphenylsulfone, ethylene diamine, hexamethylene diamine, 1,2-diamino-2 methylpropane, 3,3-diamino-n-methyldipropylamine, 1,4-diaminobutane, 1,7 diaminoheptane, 2,2,4-trimethylhexamethylene diamine, and 1,8-diaminooctane. Alternatively, Link¹ can be formed from a moiety that is a bifunctional electrophile such as diisocyanates, dicarboxylates, diesters, and dicarbonates.

### Biologically Active Agents

The Bio components are: Anti-inflammatory: non-steroidal-Oxaceprol, steroidal Enoxolone. Exemplary, non-limiting Bio components of the invention are provided in Tables 1 and 2.

**Table 1. Exemplary Pharmaceutical Molecules Used for the Synthesis of Compounds of Formula (I)**

| **Pharmaceuticals** | **Function** | **Chemical structures** |
|---|---|---|
| Amfenac | Antiinflammatory | 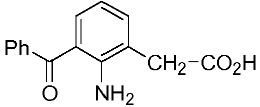 |
| Aceclofenac | Antiinflammatory | 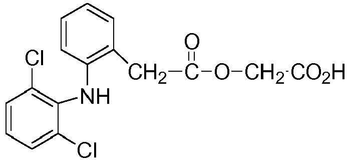 |
| Oxaceprol | Antiinflammatory | 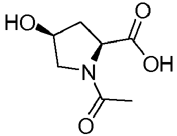 |
| Enoxolone | Antiinflammatory | 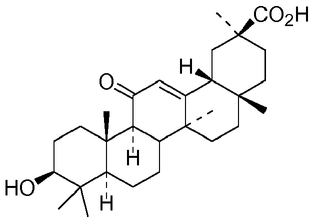 |
| Hydrocortisone | Antiinflammatory | 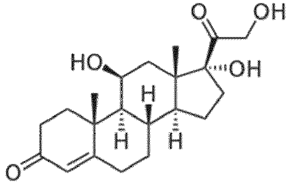 |
| Ibuprofen | Antiinflammatory | 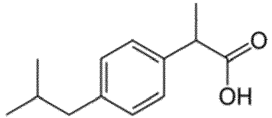 |

### Combination Therapy

In addition to one or more (e.g., two or more) compounds of the invention (e.g., the compounds of formula (I) or (I-A)), the coated surface of an article of the invention may contain an additional free biologically active agent, e.g., an antibiotic agent. Examples of antibiotic agents include: aminoglycosides, such as amikacin, apramycin, arbekacin, bambermycins, butirosin, dibekacin, dihydrostreptomycin, fortimicin(s), fradiomycin, gentamicin, ispamicin, kanamycin, micronomicin, neomycin, neomycin undecylenate, netilmicin, paromomycin, ribostamycin, sisomicin, spectinomycin, streptomycin, streptonicozid, and tobramycin; amphenicols, such as azidamfenicol, chloramphenicol, chloramphenicol palmirate, chloramphenicol pantothenate, florfenicol, and thiamphenicol; ansamycins, such as rifampin, rifabutin, rifapentine, and rifaximin; β-Lactams, such as amidinocillin, amdinocillin, pivoxil, amoxicillin, ampicillin, aspoxicillin, azidocillin, azlocillin, bacampicillin, benzylpenicillinic acid, benzylpenicillin, carbenicillin, carfecillin, carindacillin, clometocillin, cloxacillin, cyclacillin, dicloxacillin, diphenicillin, epicillin, fenbenicillin, floxicillin, hetacillin, lenampicillin, metampicillin, methicillin, mezlocillin, nafcillin, oxacillin, penamecillin, penethamate hydriodide, penicillin G benethamine, penicillin G benzathine, penicillin G benzhydrylamine, penicillin G calcium, penicillin G hydragamine, penicillin G potassium, penicillin G, procaine, penicillin N, penicillin O, penicillin V, penicillin V benzathine, penicillin V hydrabamine, penimepicycline, phenethicillin, piperacillin, pivapicillin, propicillin, quinacillin, sulbenicillin, talampicillin, temocillin and ticarcillin; carbapenems, such as imipenem; cephalosporins, such as 1-carba (dethia) cephalosporin, cefactor, cefadroxil, cefamandole, cefatrizine, cefazedone, cefazolin, cefixime, cefmenoxime, cefodizime, cefonicid, cefoperazone, ceforanide, cefotaxime, cefotiam, cefpimizole, cefpirimide, cefpodoxime proxetil, cefroxadine, cefsulodin, ceftazidime, cefteram, ceftezole, ceftibuten, ceftizoxime, ceftriaxone, cefuroxime, cefuzonam, cephacetrile sodium, cephalexin, cephaloglycin, cephaloridine, cephalosporin, cephalothin, cephapirin sodium, cephradine, pivcefalexin, cephalothin, cefaclor, cefotetan, cefprozil, loracarbef, cefetamet, and cefepime; cephamycins such as cefbuperazone, cefmetazole, cefminox, cefetan, and cefoxitin; monobactams such as aztreonam, carumonam, and tigemonan; oxacephems such as flomoxef and moxolactam; lincosamides such as clindamycin and lincomycin; macrolides such as azithromycin, carbomycin, clarithromycin, erythromycin(s) and derivatives, josamycin, leucomycins, midecamycins, miokamycin, oleandomycin, primycin, rokitamycin, rosaramicin, roxithromycin, spiramycin and troleandomycin; polypeptides such as amphomycin, bacitracin, capreomycin, colistin, enduracidin, enylomycin, fusafungine, gramicidin(s), gramicidin S, mikamycin, polymyxin, polymyxin β-methanesulfonic acid, pristinamycin, ristocetin, teicoplanin, thiostrepton, tuberactinomycin, tyrocidine, tyrothricin, vancomycin, viomycin(s), virginiamycin and zinc bacitracin; tetracyclines such as spicycline, chlortetracycline, clomocycline, demeclocycline, doxycycline, guamecycline, lymecycline, meclocycline, methacycline, minocycline, oxytetracycline, penimepicycline, pipacycline, rolitetracycline, sancycline, senociclin and tetracycline; and 2,4-diaminopyrimidines such as brodimoprim, tetroxoprim and trimethoprim; nitrofurans such as furaltadone, furazolium, nifuradene, nifuratel, nifurfoline, nifurpirinol, nifurprazine, nifurtoinol and nitrofurantoin; sulfonamides such as acetyl sulfamethoxypyrazine, acetyl sulfisoxazole, azosulfamide, benzylsulfamide, chloramine-β, chloramine-T, dichloramine-T, formosulfathiazole, N₂-formyl-sulfisomidine, N₄-β-D-glucosylsulfanilamide, mafenide, 4'-(methyl-sulfamoyl)sulfanilanilide, p-nitrosulfathiazole, noprylsulfamide, phthalylsulfacetamide, phthalylsulfathiazole, salazosulfadimidine, succinylsulfathiazole, sulfabenzamide, sulfacetamide, sulfachlorpyridazine, sulfachrysoidine, sulfacytine, sulfadiazine, sulfadicramide, sulfadimethoxine, sulfadoxine, sulfaethidole, sulfaguanidine, sulfaguanol, sulfalene, sulfaloxic acid, sulfamerazine, sulfameter, sulfamethazine, sulfamethizole, sulfamethomidine, sulfamethoxazole, sulfamethoxypyridazine, sulfametrole, sulfamidochrysoidine, sulfamoxole, sulfanilamide, sulfanilamidomethanesulfonic acid triethanolamine salt, 4-sulfanilamidosalicyclic acid, N₄-sulfanilylsulfanilamide, sulfanilylurea, N-sulfanilyl-3,4-xylamide, sulfanitran, sulfaperine, sulfaphenazole, sulfaproxyline, sulfapyrazine, sulfapyridine, sulfasomizole, sulfasymazine, sulfathiazole, sulfathiourea, sulfatolamide, sulfisomidine and sulfisoxazole; sulfones, such as acedapsone, acediasulfone, acetosulfone, dapsone, diathymosulfone, glucosulfone, solasulfone, succisulfone, sulfanilic acid, p-sulfanilylbenzylamine, p,p'-sulfonyldianiline-N,N'digalactoside, sulfoxone and thiazolsulfone; lipopeptides such as daptomycin; oxazolidones such as linezolid; ketolides such as telithromycin; and miscellaneous antibiotics such as clofoctol, hexedine, magainins, methenamine, methenamine anhydromethylene-citrate, methenamine hippurate, methenamine mandelate, methenamine sulfosalicylate, nitroxoline, squalamine, xibornol, cycloserine, mupirocin, and tuberin.

### Synthesis

Compounds of the invention may be prepared according to methods known in the art. A non-limiting example of a general synthetic procedure for preparing compounds of the invention (e.g., compounds according to formula (I) or formula (I-A)) is provided in Scheme A.

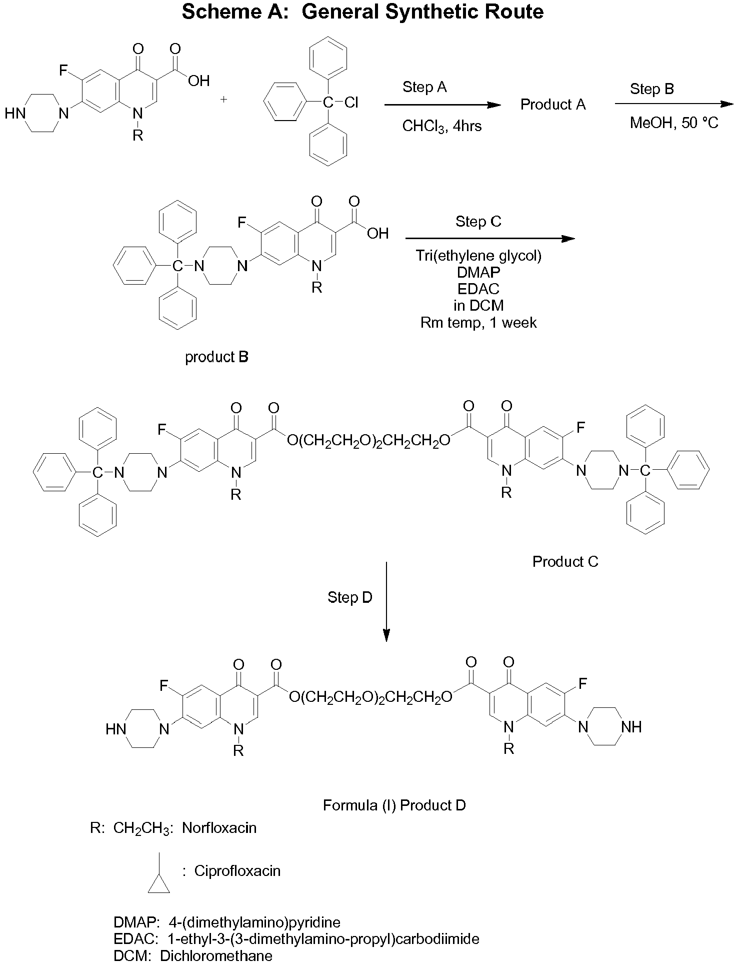

In step A, a biologically active drug, such as norfloxacin or ciprofloxacin (in the form of hydrochloride salt), is protected by a reaction between the biologically active agent and a protecting group precursor, such as trityl halide, in a suitable solvent, such as chloroform. Many other solvents may be needed depending on the solubility of the selected protecting groups and the agents forming the compound of the invention. Suitable trityl halides include trityl chloride and trityl bromide. In step B, the reaction product of step A, such as norfloxacin/ciprofloxacin with both amine and carboxylic acid groups protected with trityl group, is selectively deprotected to yield product B containing free carboxylic acid and N-tritylamine groups. In step C, the purified amine-protected fluroquinolone is coupled to both sides of a diol or diamine (in this example, triethylene glycol is used) containing an appropriate precursor. For example, the purified amine-protected fluroquinolone (Product B) is coupled to a tri(ethylene glycol) in the presence of a suitable coupling agent such as I-ethyl-3-(3- dimethylamino-propyl)carbodiimide herein denoted as EDAC and an appropriate base such as 4-(dimethylamino)pyridine herein denoted as DMAP as a catalyst. Other coupling reagents may include various carbodiimides such as CMC (1-cyclohexyl-3-(2-morpholinoethyl)carbodiimide), DCC (N,N'-dicyclohexyl- carbodiimide), DIC (Diisopropyl carbodiimide) etc, but are not limited to these. In step D, the N-trityl amine groups of the purified product C are deprotected to yield the corresponding desired product. Further synthetic details are provided in the examples.

### Shaped Articles

The compounds described herein can be used as coatings for shaped articles. Any shaped article can be coated with the compounds, compositions, and/or admixtures of the invention. For example, articles suitable for contact with bodily fluids, such as medical can be coated using the compositions described herein. The duration of contact may be short, for example, as with surgical instruments or long term use articles such as implants. The medical devices include, without limitation, catheters, guide wires, vascular stents, microparticles, electronic leads, probes, sensors, drug depots, transdermal patches, vascular patches, blood bags, orthopedics (e.g., screws and plates), hernia mesh, ophthalmological devices (i.e., punctal plug, contact lenses), vaginal slings, and tubing.

Coatings according to the invention may be used as a surface covering for an article, or, most preferably, where the polymers or admixtures are of a type capable of being formed into 1) a self-supporting structural body, 2) a film; or 3) a fiber, preferably woven or knit. The composition may comprise a surface or in whole or in part of the article, which is a biomedical device. The applications may include cardiac assist devices, tissue engineering polymeric scaffolds and related devices, cardiac replacement devices, cardiac septal patches, intra aortic balloons, percutaneous cardiac assist devices, extra-corporeal circuits, A-V fistual, dialysis components (tubing, filters, membranes, etc.), aphoresis units, membrane oxygenator, cardiac by-pass components(tubing, filters, etc.), pericardial sacs, contact lens, cochlear ear implants, sutures, sewing rings, cannulas, contraceptives, syringes, o-rings, bladders, penile implants, drug delivery systems, drainage tubes , pacemaker lead insulators, heart valves, blood bags, coatings for implantable wires, catheters, vascular stents, angioplasty balloons and devices, bandages, heart massage cups, tracheal tubes, mammary implant coatings, artificial ducts, craniofacial and maxillofacial reconstruction applications, ligaments, fallopian tubes.

The medical device can be an implanted device, or percutaneous device. Implanted devices include articles that are fully implanted in a patient, i.e., are completely internal. Percutaneous devices include items that penetrate the skin, thereby extending from outside the body into the body. Implanted devices include, without limitation, prostheses such as pacemakers, electrical leads such as pacing leads, defibrillarors, artificial hearts, ventricular assist devices, anatomical reconstruction prostheses such as breast implants, artificial heart valves, heart valve stents, pericardial patches, surgical patches, coronary stents, vascular grafts, vascular and structural stents, vascular or cardiovascular shunts, biological conduits, pledges, sutures, annuloplasty rings, stents, staples, valved grafts, dermal grafts for wound healing, orthopedic spinal implants, orthopedic pins, intrauterine devices, urinary stents, maxial facial reconstruction plating, dental implants, intraocular lenses, clips, sternal wires, bone, skin, ligaments, tendons, and combination thereof. Percutaneous devices include, without limitation, catheters or various types, cannulas, drainage tubes such as chest tubes, surgical instruments such as forceps, retractors, needles, and gloves, and catheter cuffs.

An implantable medical device as described above is generally structured from a base metallic or polymeric platform in a solid state format. The composition of the invention, either alone or as an admixture, controls the release of therapeutic agents from the device for local drug delivery applications.

The following examples, as set forth below and as summarized in **Table 3**. are put forth so as to provide those of ordinary skill in the art with a complete disclosure and description of how the methods and compounds claimed herein are performed, made, and evaluated, and are intended to be purely exemplary of the invention and are not intended to limit the scope of what the inventors regard as their invention.

**Table 3**

| Examples 1-7 and 9-27 are not according to the invention. | | |
|---|---|---|
| **Example** | **Compound** | **Description** |
| 1 | 2 | Cipro : Triethylene glycol |
| 2 | 3 | Cipro : Triethylene glycol |
| 3 | 4 | Cipro : Polyethylene glycol methyl ester |
| 4 | 5 | Cipro : Polyethylene glycol |
| 5 | 6 | Cipro : Polyethylene glycol mono methyl ether |
| 6 | 7 | Cipro : Hexane-1,2,3,4,5,6-hexol |
| 7 | 8 | Cipro : Alkoxylated Polyol |
| 8 | 9 | Hydrocortisone : Triethylene glycol |
| 9 | 10 | Cipro : Pentaerythritol ethoxylate |
| 10 | 11 | Cipro : Xylitol |
| 11 | 12 | Ofloxacin : Triethylene glycol |
| 12 | 2 | Acute Systemic Toxicity |
| 13 | 3 | Acute Systemic Toxicity |
| 14 | 2 | Intracutaneous Reactivity |
| 15 | 3 | Intracutaneous Reactivity |
| 16 | 2 | Coating |
| 17 | 3 | Coating |
| 18 | 2 and 3 | Coating |
| 19 | 2 and 3 | Gel Matrix Composition |
| 20 | 2 | Compounding |
| 21 | 2 | Heat Press |
| 22 | 2 | Drug Release in Solution |
| 23 | 3 | Drug Release in Solution |
| 24 | 2 | Accelerated Drug Release |
| 25 | 2 | Drug Release from device prototype |
| 26 | 2 | MIC/MBC |
| 27 | 3 | MIC/MBC |

### EXAMPLE 1: Synthesis and characterization of Compound 2

Ciprofloxacin HCl (1 mol) and trityl chloride (2.2 mols eqv.) were weighed in a flask and stirred in chloroform (1 L) at room temperature under N₂. Triethylamine (3.2 mols eqv.) was added dropwise into the solution and stirred at room temperature under N₂ for 4 hours.

Methanol (500 mL) was added into the reaction flask and heated to 50°C for 1.5 hours under N₂. At the end of 1.5 hour reaction, the reaction flask was cooled to room temperature. The resulting solution was washed with water (2x2L). The organic layer was dried over sodium sulphate. A small amount of methanol was added into solution and the reaction flask was placed in refrigerator overnight. The product was collected by filtration (Compound 1).

Compound 1 (2.1 mol) and DMAP (1.05 eqv.) were weighed in a flask and stirred in anhydrous dichloromethane (900 mL) under N₂ at room temperature until dissolved. Triethylene glycol (1 mol eqv.) was added dropwise into reaction flask. The reaction flask was then placed in an ice bath and solution was stirred under N₂. EDC (8.4 mol eqv.) was weighed and quickly added into the reaction flask. The reaction was allowed to proceed for 1 week at room temperature under N₂. At the end of reaction period, solvent was removed to one third of the original volume by rotary evaporator. Methanol was added into the flask and placed in -20°C freezer overnight to precipitate. Solution mixture was then filtered, and solid product was collected and dried.

Solid (1 mol) was weighed in a beaker, dichloromethane was added (100 mL), and stirred. Trifluoroacetic acid solution was prepared in water at 3.08 g/mL. Trifluoroacetic acid solution (4 mol eqv.) was added into the beaker dropwise and let stirred for few hours at room temperature. Solution mixture was then filtered and solid product was washed two times with chloroform.

In a beaker, solid was weighed, chloroform: water mixture (1.3:1 v/v) was added into the beaker, and stirred at room temperature. Saturated bicarbonate solution was prepared in water and added to solution mixture dropwise until pH 8 was reached. When desired pH is reached, solution mixture was filtered and solid was collected and dried in vacuum oven for 2 days.

Compound 2: HPLC (mobile phase H2O/TFA and MeCN/TFA)19.857 min. Sodium analysis = 1240 ppm. ¹H NMR (300 MHz, dDMSO) δ (ppm) 1.06-1.22(C**H**₂-CH, ciprofloxacin), 3.32 (C**H₂**-NH, ciprofloxacin), 3.41(C**H₂**-N-, ciprofloxacin), 3.56 (C**H**-, ciprofloxacin), 3.64 (O-C**H₂**-C**H₂**-O, TEG), 3.72 (C**H₂**-O, TEG), 4.24 (C**H₂**-OOC, TEG), 7.33 (**H**C=C-N, ciprofloxacin), 7.49 (**H**C=C-F, ciprofloxacin), 8.31 (N-C(**H**)=C(CO)-COO-, ciprofloxacin). ¹⁹F NMR (300 MHz, dDMSO) δ (ppm) -124.8 (HC=C-**F**, ciprofloxacin)

### EXAMPLE 2: Synthesis and characterization of Compound 3

Compound 1 (1 mol) and DMAP (0.505 mol eqv.) were weighed in a flask and stirred in anhydrous dichloromethane (900 mL) under N₂ at room temperature until dissolved. Triethylene glycol (10 mol eqv.) was added dropwise into reaction flask. The reaction flask was placed in an ice bath and solution was stirred under N₂. EDC (4.1 mol eqv.) was weighed and quickly added into the reaction flask. The reaction was allowed to proceed for 1 week at room temperature under N₂. At the end of reaction period, solvent was removed to one third the original volume by rotary evaporator. Methanol was added into the flask and placed in -20°C freezer overnight to precipitate. Solution mixture was then filtered, and solid product was collected and dried.

Solid was dissolved in chloroform (1.5% w/v) and loaded onto silica resin column packed in chloroform (50:1 w/w silica:solid product). Impurities were eluted through the column using chloroform as mobile phase and then mobile phase was switched to 5% methanol in chloroform to elute product. Fractions corresponding to product were collected and solvent was removed completely by rotary evaporator to give solid product.

Solid (1 mol) was weighed in a beaker, dichloromethane was added (20 mL), and mixture was stirred. Trifluoroacetic acid solution was prepared in water at 3.08 g/mL. Trifluoroacetic acid solution (2 mol eqv.) was added into the beaker dropwise and solution was stirred for 0.5-1 hour at room temperature. Water (40 ml) was added to solution, mixed well, and the aqueous phase was collected. The water extraction was repeated on organic phase and the two aqueous phases were combined. Saturated bicarbonate solution was prepared in water and added to solution mixture dropwise until pH 8 was reached. When desired pH was reached, solution was frozen at -20°C and product was recovered by lyophilization.

Compound 3: HPLC (mobile phase H2O/TFA and MeCN/TFA) 19.090 min. Sodium analysis = 1870 ppm. Mass spectroscopy (m/z) 464.2. ¹H NMR (300 MHz, dDMSO) δ (ppm) 1.06-1.24(C**H₂**-CH, ciprofloxacin), 3.14 (CH₂-C**H₂**-O-C**H₂**-CH₂, TEG), 3.30 (C**H₂**-NH, ciprofloxacin), 3.41(C**H₂**-N-, ciprofloxacin), 3.56 (C**H**- and C**H₂**-OH, ciprofloxacin and TEG, respectively), 3.68 (O-C**H₂**-C**H₂**-O and C**H₂**-O, TEG), 4.27 (C**H₂**-OOC, TEG), 7.42 (**H**C=C-N, ciprofloxacin), 7.77 (**H**C=C-F, ciprofloxacin), 8.43 (N-C(**H**)=C(CO)-COO-, ciprofloxacin). ¹⁹F NMR (300 MHz, dDMSO) δ (ppm) -124.5 (HC=C-**F**, ciprofloxacin).

### EXAMPLE 3: Synthesis and characterization of Compound 4

Compound 1 (1.1 mol) and DMAP (0.53 mol eqv.) was weighed in a flask and stirred in anhydrous dichloromethane (870 mL) under N₂ at room temperature until dissolved. Poly(ethylene glycol) methyl ester or Poly(ethylene glycol) methyl ether (1 mol eqv.) was dissolved in dichloromethane (30 mL) and added dropwise into reaction flask. The reaction flask was then placed in an ice bath and solution was stirred under N₂. EDC (4.1 mol eqv.) was weighed and quickly added into the reaction flask. The reaction was allowed to proceed for 10 days at room temperature under N₂. At the end of reaction period, solvent was removed to one third of the original volume by rotary evaporator. Methanol was added into the flask and placed in -20°C freezer overnight to precipitate. Solution mixture was then filtered, and solid product was collected and dried.

Solid (1 mol) was weighed in a beaker, dichloromethane was added (100 mL), and stirred. Trifluoroacetic acid solution was prepared in water at 3.08 g/mL. Trifluoroacetic acid solution (2 mol eqv.) was added into the beaker dropwise and let stirred for few hours at room temperature. Solution mixture was then filtered and solid product was washed three times with dichloromethane.

In a beaker, solid was weighed, dichloromethane: water mixture (5:1 v/v) is added into the beaker, and stirred at room temperature. Saturated bicarbonate solution was prepared in water and added to solution mixture dropwise until pH 8 is reached. When desired pH was reached, solution mixture was filtered and solid was collected and dried in vacuum oven for 2 days.

Characterization was completed using TLC, HPLC, ¹H NMR analysis.

### EXAMPLE 4: Synthesis and characterization of Compound 5

Compound 1 (2.1 mol) and DMAP (1.05 eqv.) were weighed in a flask and stirred in anhydrous dichloromethane (870 mL) under N₂ at room temperature until dissolved. Poly(ethylene glycol) (1 mol eqv.) was dissolved in dichloromethane (30 mL) and added dropwise into reaction flask. The reaction flask was then placed in an ice bath and solution is stirred under N₂. EDC (8.4 mol eqv.) was weighed and quickly added into the reaction flask. The reaction was allowed to proceed for 10 days at room temperature under N₂. At the end of reaction period, solvent was removed to one third of the original volume by rotary evaporator. Methanol was added into the flask and placed in -20°C freezer overnight to precipitate. Solution mixture was then filtered, and solid product was collected and dried.

Solid (1 mol) was weighed in a beaker, dichloromethane was added (100 mL), and stirred. Trifluoroacetic acid solution was prepared in water at 3.08 g/mL. Trifluoroacetic acid solution (4 mol eqv.) was added into the beaker dropwise and let stirred for few hours at room temperature. Solution mixture was then filtered and solid product was washed three times with dichloromethane.

In a beaker, solid was weighed, dichloromethane: water mixture (5:1 v/v) was added into the beaker, and stirred at room temperature. Saturated bicarbonate solution was prepared in water and added to solution mixture dropwise until pH 8 is reached. When desired pH was reached, solution mixture was filtered and solid was collected and dried in vacuum oven for 2 days.

Characterization was completed using TLC, HPLC, ¹H NMR analysis.

### EXAMPLE 5: Synthesis and characterization of Compound 6

Compound 1 (2.1 mol) and DMAP (1.05 eqv.) were weighed in a flask and stirred in anhydrous dichloromethane (850 mL) under N₂ at room temperature until dissolved. Polyethylene glycol mono methyl ether (1 mol eqv.) was dissolved in dichloromethane (50 mL) and added into reaction flask dropwise. The reaction flask was then placed in an ice bath and solution is stirred under N₂. EDC (8.4 mol eqv.) was weighed and quickly added into the reaction flask. The reaction was allowed to proceed for 10 days at room temperature under N₂. At the end of reaction period, solvent was removed to one third of the original volume by rotary evaporator. Methanol was added into the flask and placed in -20°C freezer overnight to precipitate. Solution mixture was then filtered, and solid product was collected and dried.

Solid (1 mol) was weighed in a beaker, dichloromethane was added (100 mL), and stirred. Trifluoroacetic acid solution was prepared in water at 3.08 g/mL. Trifluoroacetic acid solution (4 mol eqv.) was added into the beaker dropwise and let stirred for few hours at room temperature. Solution mixture was then filtered and solid product was washed three times with dichloromethane.

In a beaker, solid was weighed, dichloromethane: water mixture (5:1 v/v) was added into the beaker, and stirred at room temperature. Saturated bicarbonate solution was prepared in water and added to solution mixture dropwise until pH 8 was reached. When desired pH was reached, solution mixture was filtered and solid was collected and dried in vacuum oven for 2 days.

Characterization was completed using TLC, HPLC, ¹H NMR analysis.

### EXAMPLE 6: Synthesis and characterization of Compound 7

Compound 1 (6.1 mol) and DMAP (3.2 mol eqv.) were weighed in a flask and stirred in anhydrous dichloromethane (900 mL) under N₂ at room temperature until dissolved. Hexane-1,2,3,4,5,6-hexol (1 mol eqv.) was added dropwise into reaction flask. The reaction flask was then placed in an ice bath and solution is stirred under N₂. EDC (24.4 mol eqv.) was weighed and quickly added into the reaction flask. The reaction was allowed to proceed for 10 days at room temperature under N₂. At the end of reaction period, solvent was removed to one third of the original volume by rotary evaporator. Methanol was added into the flask and placed in -20°C freezer overnight to precipitate. Solution mixture was then filtered, and solid product was collected and dried.

Solid (1 mol) was weighed in a beaker, dichloromethane was added (100 mL), and stirred. Trifluoroacetic acid solution was prepared in water at 3.08 g/mL. Trifluoroacetic acid solution (12 mol eqv.) was added into the beaker dropwise and let stirred for few hours at room temperature. Solution mixture was then filtered and solid product was washed three times with dichloromethane.

In a beaker, solid was weighed, dichloromethane: water mixture (5:1 v/v) was added into the beaker, and stirred at room temperature. Saturated bicarbonate solution was prepared in water and added to solution mixture dropwise until pH 8 was reached. When desired pH is reached, solution mixture was filtered and solid was collected and dried in vacuum oven for 2 days.

Characterization was completed using TLC, HPLC, ¹H NMR analysis.

### EXAMPLE 7: Synthesis and characterization of Compound 8

Compound 1 (3.1 mol) and DMAP (1.58 mol eqv.) were weighed in a flask and stirred in anhydrous dichloromethane (900 mL) under N₂ at room temperature until dissolved. Alkoxylated Polyol (1 mol eqv.) was added dropwise into reaction flask. The reaction flask was then placed in an ice bath and solution was stirred under N₂. EDC (12.4 mol eqv.) was weighed and quickly added into the reaction flask. The reaction was allowed to proceed for 10 days at room temperature under N₂. At the end of reaction period, solvent was removed to one third of the original volume by rotary evaporator. Methanol was added into the flask and placed in -20°C freezer overnight to precipitate. Solution mixture was then filtered, and solid product was collected and dried.

Solid (1 mol) was weighed in a beaker, dichloromethane was added (100 mL), and stirred. Trifluoroacetic acid solution was prepared in water at 3.08 g/mL. Trifluoroacetic acid solution (6 mol eqv.) was added into the beaker dropwise and let stirred for few hours at room temperature. Solution mixture was then filtered and solid product was washed three times with dichloromethane.

In a beaker, solid was weighed, dichloromethane: water mixture (5:1 v/v) was added into the beaker, and stirred at room temperature. Saturated bicarbonate solution was prepared in water and added to solution mixture dropwise until pH 8 was reached. When desired pH is reached, solution mixture is filtered and solid was collected and dried in vacuum oven for 2 days.

Characterization was completed using TLC, HPLC, ¹H NMR analysis.

### EXAMPLE 8: Synthesis and characterization of Compound 9

Hydrocortisone (1 mol) and triethyleneamine (0.5 mols eqv.) were weighed in a flask and stirred in dichloromethane at room temperature under N2. Bis activated carbonate (2 mols eqv.) was added into the solution and stirred at room temperature under N2 overnight. Purification was performed using crystallization and column chromatography.

Characterization was completed using TLC, HPLC, ¹H NMR analysis.

### EXAMPLE 9: Synthesis and characterization of Compound 10

Compound 1 (4.1 mol) and DMAP (2.1 mol eqv.) were weighed in a flask and stirred in anhydrous dichloromethane (870 mL) under N₂ at room temperature until dissolved. Pentaerythritol ethoxylate (1 mol eqv.) was dissolved in dichloromethane (30 mL) and added dropwise into reaction flask. The reaction flask was then placed in an ice bath and solution was stirred under N₂. EDC (16.4 mol eqv.) was weighed and quickly added into the reaction flask. The reaction was allowed to proceed for 10 days at room temperature under N₂. At the end of reaction period, solvent was removed to one third of the original volume by rotary evaporator. Methanol was added into the flask and placed in -20°C freezer overnight to precipitate. Solution mixture was then filtered, and solid product was collected and dried.

Solid (1 mol) was weighed in a beaker, dichloromethane was added (100 mL), and stirred. Trifluoroacetic acid solution was prepared in water at 3.08 g/mL. Trifluoroacetic acid solution (8 mol eqv.) was added into the beaker dropwise and let stirred for few hours at room temperature. Solution mixture was then filtered and solid product was washed three times with dichloromethane.

In a beaker, solid was weighed, dichloromethane: water mixture (5:1 v/v) was added into the beaker, and stirred at room temperature. Saturated bicarbonate solution was prepared in water and added to solution mixture dropwise until pH 8 was reached. When desired pH was reached, solution mixture was filtered and solid was collected and dried in vacuum oven for 2 days.

Characterization was completed using TLC, HPLC, ¹H NMR analysis.

### EXAMPLE 10: Synthesis and characterization of Compound 11

Compound 1 (5.1 mol) and DMAP (2.63 mol eqv.) are weighed in a flask and stirred in anhydrous dichloromethane (870 mL) under N₂ at room temperature until dissolved. Xylitol (1 mol eqv.) is added dropwise into reaction flask. The reaction flask is then placed in an ice bath and solution is stirred under N₂. EDC (20.4 mol eqv.) is weighed and quickly added into the reaction flask. The reaction is allowed to proceed for 10 days at room temperature under N₂. At the end of reaction period, solvent is removed to one third of the original volume by rotary evaporator. Methanol is added into the flask and placed in -20°C freezer overnight to precipitate. Solution mixture is then filtered, and solid product is collected and dried.

Solid (1 mol) is weighed in a beaker, dichloromethane is added (100 mL), and stirred. Trifluoroacetic acid solution is prepared in water at 3.08 g/mL. Trifluoroacetic acid solution (10 mol eqv.) is added into the beaker dropwise and let stirred for few hours at room temperature. Solution mixture is then filtered and solid product is washed three times with dichloromethane.

In a beaker, solid is weighed, dichloromethane: water mixture (5:1 v/v) is added into the beaker, and stirred at room temperature. Saturated bicarbonate solution is prepared in water and added to solution mixture dropwise until pH 8 is reached. When desired pH is reached, solution mixture is filtered and solid is collected and dried in vacuum oven for 2 days.

Characterization are performed using TLC, HPLC, ¹H NMR analysis.

### EXAMPLE 11: Synthesis and characterization of Compound 12

Ofloxacin (2.1 mol) and DMAP (1.05 eqv.) were weighed in a flask and stirred in anhydrous dichloromethane (900 mL) under N2 at room temperature until dissolved. Triethylene glycol (1 mol eqv.) was added into reaction flask. The reaction flask was then placed in an ice bath and solution was stirred under N2. EDC (8.4 mol eqv.) was weighed and quickly added into the reaction flask. The reaction was allowed to proceed for 1 week at room temperature under N2. The resulting solution was washed with water (2x2L). The organic layer was dried over sodium sulphate. Mixture solution was filtered and filtrate was collected. Dichloromethane was removed to approximately 20% of the original volume by rotary evaporator. Acetone was added into the flask at 1:1 (v/v) ratio and placed in -20°C freezer overnight to precipitate. Precipitate was then filtered, collected and dried.

Compound 12: HPLC (mobile phase H2O/TFA and MeCN/TFA) 19.678 min and 19.868 min. Mass spectroscopy (m/z) 836.4. 1H NMR (300 MHz, CDCI3) δ (ppm) 1.56 (CH3-CH, ofloxacin), 2.37 (CH3-N, ofloxacin), 2.56 (-O-CH2-CH (CH3), ofloxacin), 3.34 (-N-CH2-CH2-N-, ofloxacin), 3.77 (O-CH2-CH2-O, TEG), 3.85 (-CH2-O, TEG), 4.38 (CH-C(CH3), ofloxacin), 4.84 (CH2-OOC, TEG), 7.21 (HC=C-F, ofloxacin), 8.22 (N-C(H)=C(CO)-COO-, ofloxacin).

### EXAMPLE 12: Acute Systemic Toxicity Testing of Compound 2

Compound 2 was dissolved in PBS (4x10⁻⁵ - 9.7x10⁻¹ mg/mL) and tested for Acute Systemic Toxicity following ISO 10993-11. A single injection dose of 50 mL/kg per mouse (∼1 mL) was administered to 5 mice per test sample. Mice were observed immediately post injection and at 4, 24, 48 and 72h for signs of toxicity compared to control. Higher dosage demonstrated no signs of toxicity.

### EXAMPLE 13: Acute Systemic Toxicity Testing of Compound 3

Compound 3 was dissolved in PBS (8x10⁻⁴ - 8x10⁻² mg/mL) and tested for Acute Systemic Toxicity following ISO 10993-11. A single injection dose of 50 mL/kg per mouse (∼1 mL) was administered to 5 mice per test sample. Mice were observed immediately post injection and at 4, 24, 48 and 72h for signs of toxicity compared to control. Compound 3 at all concentrations showed no signs of toxicity.

### EXAMPLE 14: Intracutaneous Reactivity Testing of Compound 2

Compound 2 was dissolved in PBS (9.2x10⁻¹ mg/mL) and tested for Intracutaneous Reactivity in accordance with ISO 10993-10: 2010 Standard, Biological Evaluation of Medical Devices, Part 10: Tests for Irritation and Skin Sensitization, Pages 11-14. Each rabbit received five sequential 0.2 mL intracutaneous injections along either side of the dorsal midline with the test article on one side and the control on the other. Three New Zealand rabbits were used per sample and control. The injection sites were observed and scored for erythema (redness) and edema (swelling) after 24, 48, and 72 h on a scale of 1 to 4. Compound 2 showed no signs of irritation and was deemed a non-irritant.

### EXAMPLE 15: Intracutaneous Reactivity Testing of Compound 3

Compound 3 was dissolved in PBS (5.4x10⁻² mg/mL) and tested for Intracutaneous Reactivity in accordance with ISO 10993-10: 2010 Standard, Biological Evaluation of Medical Devices, Part 10: Tests for Irritation and Skin Sensitization, Pages 11-14. Each rabbit received five sequential 0.2 mL intracutaneous injections along either side of the dorsal midline with the test article on one side and the control on the other. Three New Zealand rabbits were used per sample and control. The injection sites were observed and scored for erythema (redness) and edema (swelling) after 24, 48, and 72 h on a scale of 1 to 4. Compound 3 showed no signs of irritation and was deemed a non-irritant.

### EXAMPLE 16: Coating Compound 2 on Polymeric Surfaces

Dacron meshes (TDA PETNF203) at 0.5 cm x 2 cm and Hernia meshes were dip coated with a range of Compound 2 solutions (1 - 30 mg/mL) in various solvents (DMF, DMSO, Methanol). Further increases in loading (up to ∼13 mg) were achieved by dipping the Dacron meshes in solution multiple times at 30 mg/ml with drying periods between each dip. Loading was determined by stripping samples for 6h in DMF and analyzing by RP-HPLC using established protocols. SEM analysis of coated meshes in DMF showed a smooth coating with limited webbing (Figure 1). No changes in chemical structure were observed after stripping coated sample in d₆-DMSO for 1 h and analyzing by ¹H NMR.

Dacron meshes were also coated with Compound 2 and Chlrohexidine (CHX) in various solvents. SEM analysis of coated meshes showed a smooth coating. The release profile and biological efficacy of Compound 2 (Cip) was not impacted by the presence of CHX.

### EXAMPLE 17: Coating Compound 3 on Polymeric Surfaces

Dacron mesh (TDA PETNF203) at 0.5 cm x 2 cm was dip coated with Compound 3 and dried at room temperature under vacuum. SEM of coated mesh showed a smooth coating with limited webbing (Figure 2).

### EXAMPLE 18: Coating Compound 2 and 3 on Metallic Surfaces

Stainless steel coupons and orthopedic screws were dipped once for 30s in either a 10 mg/mL solution of Compound 2, Compound 3, ciprofloxacin HCl in DMF, or DMF alone as control. Compound 2 and ciprofloxacin HCl samples were dried in a 50°C flow oven for 5h while Compound 3 was dried at 60°C. After drying, visual observations were made using light microscopy (Figure 3). Coupons with ciprofloxacin HCl had a white uneven coating, while those coated with Compounds 2 and 3 were clear.

### EXAMPLE 22: Drug release from Compound 2 in Solution

Compound 2 was dissolved in PBS (pH 7.4) at 0.1 mg/ml and placed in incubator at 37°C. At each time point (0, 1, 3, 7, 14, 21, & 28 days), solution was removed from incubator and analyzed for the drug by RP-HPLC using established protocols (Figure 6). After 28 days, -8% total drug was released from Compound 2, demonstrating slow and sustained release under these conditions. The release profile of compound 2 was also evaluated in a device prototype assembly using bovine serum, blood (porcine) or a gel matrix.

### EXAMPLE 23: Drug release from Compound 3 in Solution

Compound 3 was dissolved in PBS (pH 7.4) at 0.1 mg/ml and placed in incubator at 37°C. At each time point (0, 1, 3, 7, 14, 21, & 28 days), solution was removed from incubator and analyzed for drug by RP-HPLC using established protocols. A linear increase in the drug concentration was observed with time out to at least 28 days (Figure 7).

### EXAMPLE 24: Accelerated drug release from Compound 2

Compound 2 was prepared at 10 mg/ml in 0.1N HCl (final pH ∼4), 0.1N NaOH (final pH ∼10), and PBS (final pH ∼7). Samples were incubated at 37°C in acidic, basic, or neutral conditions. At each time point, the drug concentration was quantified by RP-HPLC. Faster drug release was demonstrated under acidic and basic conditions: basic pH (100% release in less than 1 day) >> acidic pH (∼71% after 7 days) >> neutral pH (∼2% after 7 days).

### EXAMPLE 25: Drug Release from Compound 2 Coated onto Dacron

Compound 2 was coated onto 0.5 cm x 2 cm Dacron meshes and dried at 60°C overnight. Coated meshes were assembled on catheters. Drug release from the coated meshes pre- and post-assembly was carried out in 2 ml PBS (pH 7.4) at 37°C for 24h. drug released into solution was quantified by RP-HPLC.

### EXAMPLE 26: MIC & MBC Determination for Compound 2 and drug Released from Compound 2

The antimicrobial efficacy of Compound 2 and drug released from Compound 2 was investigated using a standard broth microdilution method. The minimum inhibitory concentration (MIC) and minimum bactericidal concentration (MBC) were investigated for Compound 2, drug released from Compound 2, triethylene glycol (TEG), Ciprofloxacin hydrochloride (Cipro®HCl), chlorhexidine diacetate (CHX-A), and Compound 2 + CHX-A. The MIC is defined as the lowest concentration of an antimicrobial agent that will inhibit visible growth of a microorganism after overnight incubation. The MBC is defined as the lowest concentration of an antimicrobial agent required to kill 99.9% of a microorganism population. This study was carried out using two gram positive bacteria, *E. faecalis* (ATCC 29212) and S. *aureus* (ATCC 25923), and two gram negative bacteria, *E. coli* (ATCC 25922) and P. *aeruginosa* (ATCC 27853). The test samples were prepared using 2-fold dilutions per well and covered a concentration range at least 2 dilutions above and 2 dilutions below literature MIC values for the microorganisms tested. **Table 4** summarizes the results of the study for S. *aureus.* Compound 2 did not show any antimicrobial activity at the highest concentration tested. Drug released from Compound 2 showed antimicrobial activity consistent with both the Cipro®HCl controls and literature values. No antimicrobial activity was observed with TEG linker at the highest concentration tested. Testing Compound 2 in combination with CHX-A did not affect the activity of CHX-A, demonstrating the potential for additive or combination therapy with a second antimicrobial agent. Similar results were observed for the other microorganisms tested.

**Table 4: Experimental and literature MIC & MBC values for Compound 2 against S. aureus**

| **Samples** | | **Tested Concentration Range (ug/ml)** | **MIC (ug/ml)** | | **MBC (ug/ml)** | |
|---|---|---|---|---|---|---|
| | | | **Experimental** | **Literature** | **Experimental** | **Literature** |
| Compound 2 | | 78 - 0.076 | >78 | n/d | >78 | n/d |
| TEG | | 100 - 0.098 | >100 | | >100 | |
| Cipro*HCl | | 4 - 0.004 | 025 - 05 | 0.5 | 0.5 - 1 | 0.9 |
| Drug Released from Compound 2 | | 4 - 0.004 | 0.5 | | 0.5 | |
| CHX-A | | 128 - 0.125 | 1 | 0.9 | 1-4 | 3.9 |
| Compound 2 + CHX-A | 2 | 78 - 0.076 | - | | - | |
| | CHX-A | 128 - 0.125 | 1 | | 2 | |

### EXAMPLE 27: MIC & MBC Determination for Compound 3 and Drug Released from Compound 3

The antimicrobial efficacy of Compound 3 and drug released from Compound 3 investigated using a standard broth microdilution method. The MIC and MBC were investigated for Compound 3, drug released from Compound 3, TEG, Cipro®HCl, CHX-A, and Compound 3 + CHX-A. This study was carried out using two gram positive bacteria, *E. faecalis* (ATCC 29212) and *S. aureus* (ATCC 25923), and two gram negative bacteria, *E. coli* (ATCC 25922) and *P. aeruginosa* (ATCC 27853). The test samples were prepared using 2-fold dilutions per well and covered a concentration range at least 2 dilutions above and 2 dilutions below literature MIC values for the microorganisms tested. **Table 5** summarizes the results of the study for *S. aureus.* Compound 3 did not show any antimicrobial activity at the highest concentration tested. Drug released from compound 3 showed antimicrobial activity consistent with both the Cipro®HCl controls and literature values. Testing Compound 3 in combination with CHX-A did not affect the activity of CHX-A, demonstrating the potential for additive or combination therapy with a second antimicrobial agent. Similar results were observed for the other microorganisms tested.

**Table 5: Experimental and literature MIC & MBC values for Compound 3 against S. aureus**

| **Samples** | | **Tested Concentration Range (ug/ml)** | **MIC** | | **MBC (ug/ml)** | |
|---|---|---|---|---|---|---|
| | | | **Experimental** | **Literature** | **Experimental** | **Literature** |
| Compound 3 | | 55.5 - 0.054 | >55.5 | n/d | >55.5 | n/d |
| TEG | | 100 - 0.098 | >100 | | >100 | |
| Cipro*HCl | | 4 - 0.004 | 0.25 - 0.5 | 0.5 | 0.5 - 1 | 0.9 |
| Cipro Released from Compound 3 | | 4 - 0.004 | 0.5 | | 1 | |
| CHX-A | | 128 - 0.125 | 1 | 0.9 | 1 - 4 | 3.9 |
| Compound 3 + CHX-A | 3 | 555 - 0054 | - | | - | |
| | CHX-A | 128 - 0.125 | 1 | | 8 | |

## Claims

1. An article comprising a coated surface, wherein said coated surface comprises a compound having a structure according to formula (I):
Bio¹-Link¹-(Bio²-R¹)ₘ (I),
or a pharmaceutically acceptable salt thereof, wherein
Bio¹ and each Bio² are formed from a biologically active agent;
m is 1, 2, 3, 4, or 5;
wherein each Bio² comprises a covalent bond to Link¹;
R¹ is absent;
Link¹ is an oligomeric organic, organosilicon, or organosulfone segment having a molecular weight between 60 and 2000 Daltons;
wherein said article is an implantable or a percutaneous medical device; and
wherein said biologically active agent is an anti-inflammatory agent.

2. The article of claim 1, wherein said compound has a structure according to formula (I-A),
Bio¹-Link¹-Bio²-R¹ (I-A),
or a pharmaceutically acceptable salt thereof, wherein
both Bio¹ and Bio² are formed from said biologically active agent;
R¹ is absent; and
Link¹ is an oligomeric organic, organosilicon, or organosulfone segment having a molecular weight between 60 and 2000 Daltons.

3. The article of claim 1 or 2, wherein said anti-inflammatory agent is selected from the group consisting of amfenac, aceclofenac, oxaceprol, enoxolone, hydrocortisone, and ibuprofen.

4. The article of any one of claims 1 to 3, wherein Link¹ is formed from a diol, a diamine, or an α,ω-aminoalcohol.

5. The article of any one of claims 1 to 3, wherein Link¹ is formed from a polyethylene oxide having terminal amino or hydroxyl groups, and wherein Link¹ comprises 1-20 ethylene oxide repeating units.

6. The article of any one of claims 1 to 3, wherein Link¹ is formed from a compound selected from the group consisting of: ethylene glycol; butane diol; hexane diol; hexamethylene diol; 1,5-pentanediol; 2,2-dimethyl-1,3 propanediol; 1,4-cyclohexane diol; 1,4-cyclohexanedimethanol; tri(ethylene glycol); poly(ethylene glycol), where the molecular weight is between 100 and 2000 Daltons; poly(ethylene oxide) diamine, where the molecular weight is between 100 and 2000 Daltons; lysine esters; silicone diols; silicone diamines; polyether diols; polyether diamines; carbonate diols; carbonate diamines; dihydroxy vinyl derivatives; dihydroxydiphenylsulfone; ethylene diamine; hexamethylene diamine; 1,2-diamino-2-methylpropane; 3,3-diamino-n-methyldipropylamine; 1,4-diaminobutane; 1, 7-diaminoheptane; and 1,8-diaminooctane.

7. The article of any one of claims 1 to 3, wherein Link¹ is formed from tri(ethylene glycol).

8. The article of any one of claims 1 to 3, wherein Link¹ is formed from a dicarboxylic compound or a diisocyanate.

9. The article of any one of claims 1 to 8, wherein said coating comprises a second compound having a structure according to formula (I) or formula (I-A), wherein each Bio¹, Link¹, and Bio² is as defined in any one of claims 1 to 6; or
wherein said article comprises a mixture of two or more compounds according to formula (I) and/or formula (I-A).

10. The article of any one of claims 1 to 9, wherein said coating is substantially free of any biologically active agent used to form Bio¹ and/or Bio² that is not included in a compound according to formula (I) or formula (I-A); or
wherein said compound according to formula (I) has reduced biological activity compared to the biologically active agent used to form Bio¹ and/or Bio²; preferably, wherein said compound according to formula (I) or formula (I-A) has 0%-20% of the biological activity of the biologically active agent used to form Bio¹ and/or Bio².

11. The article of any one of claims 1 to 10, wherein said coated surface comprises a pharmaceutically acceptable salt of the compound according to formula (I) or formula (I-A); preferably wherein said pharmaceutically acceptable salt is the trifluoroacetate or the hydrochloride salt.

12. The article of any one of claims 1 to 11, wherein said article is an implantable device selected from: prostheses pacemakers, electrical leads, defibrillators, artificial hearts, ventricular assist devices, anatomical reconstruction prostheses, artificial heart valves, heart valve stents, pericardial patches, surgical patches, coronary stents, vascular grafts, vascular and structural stents, vascular or cardiovascular shunts, biological conduits, pledges, sutures, annuloplasty rings, stents, staples, valved grafts, dermal grafts for wound healing, orthopedic spinal implants, orthopedic devices, ophthalmic implants, intrauterine devices, stents, maxial facial reconstruction plating, dental implants, intraocular lenses, clips, sternal wires, bone, skin, ligaments, sutures, hernia mesh, tendons, and combinations thereof.

13. The article of any one of claims 1 to 11, wherein said article is a percutaneous device selected from the group consisting of: catheters, cannulas, drainage tubes, and surgical instruments; preferably, wherein said percutaneous device is a surgical instrument selected from the group consisting of forceps, retractors, needles, and catheter cuffs.

14. The article of any one of claims 1 to 13, wherein the coating on said coated surface has a thickness between 0.5 to 120 µm.

15. A method for producing the article of claim 1, said method comprising contacting the surface of an article with a composition, said composition comprising:
(a) the compound as set forth in any one of claims 1 to 8 preferably, wherein the concentration of said compound in said composition is between 0.05-150 mg/mL, and
(b) a suitable medium in which the compound of (a) is soluble; preferably wherein said suitable medium is an organic solvent or aqueous solvent; preferably, wherein said suitable medium is tetrahydrofuran or N,N-dimethylformamide.

## Patentansprüche

1. Gegenstand, umfassend eine beschichtete Oberfläche, wobei die beschichtete Oberfläche eine Verbindung umfasst, die eine Struktur gemäß Formel (I) aufweist:
Bio¹-Link¹-(Bio²-R¹)ₘ (I),
oder ein pharmazeutisch verträgliches Salz davon, wobei
Bio¹ und jedes Bio² aus einem biologisch aktiven Wirkstoff gebildet sind;
m 1, 2, 3, 3, 4 oder 5 ist;
wobei jedes Bio² eine kovalente Bindung zu Link¹ umfasst;
R¹ fehlt;
Link¹ ein oligomer organisches, siliziumorganisches oder organosulfonisches Segment ist, das ein Molekulargewicht zwischen 60 und 2000 Dalton aufweist;
wobei der Gegenstand eine implantierbare oder eine perkutane medizinische Vorrichtung ist; und
wobei der biologisch aktive Wirkstoff ein entzündungshemmender Wirkstoff ist.

2. Gegenstand nach Anspruch 1, wobei die Verbindung eine Struktur nach Formel (I-A) aufweist,
Bio¹-Link¹-Bio²-R¹ (I-A),
oder ein pharmazeutisch verträgliches Salz davon, wobei
beide Bio¹ und Bio² aus dem biologisch aktiven Wirkstoff gebildet sind;
R¹ fehlt; und
Link¹ ein oligomer organisches, siliziumorganisches oder organosulfonisches Segment ist, das ein Molekulargewicht zwischen 60 und 2000 Dalton aufweist.

3. Gegenstand nach Anspruch 1 oder 2, wobei der entzündungshemmende Wirkstoff ausgewählt ist aus der Gruppe bestehend aus Amfenac, Aceclofenac, Oxaceprol, Enoxolon, Hydrocortison und Ibuprofen.

4. Gegenstand nach einem der Ansprüche 1 bis 3, wobei Link¹ aus einem Diol, einem Diamin oder einem α,ω-Aminoalkohol gebildet ist.

5. Gegenstand nach einem der Ansprüche 1 bis 3, wobei Link¹ aus einem Polyethylenoxid gebildet ist, das terminale Amino- oder Hydroxylgruppen aufweist, und wobei Link¹ 1-20 Ethylenoxid-Wiederholungseinheiten umfasst.

6. Gegenstand nach einem der Ansprüche 1 bis 3, wobei Link¹ aus einer Verbindung gebildet ist, die ausgewählt ist aus der Gruppe bestehend aus: Ethylenglykol; Butandiol; Hexandiol; Hexamethylendiol; 1,5-Pentandiol; 2,2-Dimethyl-1,3-propandiol; 1,4-Cyclohexandiol; 1,4-Cyclohexandimethanol; Tri(ethylenglykol); Poly(ethylenglykol), wobei das Molekulargewicht zwischen 100 und 2000 Dalton liegt; Poly(ethylenoxid)diamin, wobei das Molekulargewicht zwischen 100 und 2000 Dalton liegt; Lysinester; Silikondiole; Silikondiamine; Polyetherdiole; Polyetherdiamine; Carbonatdiole; Carbonatdiamine; Dihydroxyvinylderivate; Dihydroxydiphenylsulfon; Ethylendiamin; Hexamethylendiamin; 1,2-Diamino-2-Methylpropan; 3,3-Diamino-n-Methyldipropylamin; 1,4-Diaminobutan; 1,7-Diaminoheptan; und 1,8-Diaminooctan.

7. Gegenstand nach einem der Ansprüche 1 bis 3, wobei Link¹ aus Tri(ethylenglykol) gebildet ist.

8. Gegenstand nach einem der Ansprüche 1 bis 3, wobei Link¹ aus einer Dicarbonsäureverbindung oder einem Diisocyanat gebildet ist.

9. Gegenstand nach einem der Ansprüche 1 bis 8, wobei die Beschichtung eine zweite Verbindung umfasst, die die Struktur nach Formel (I) oder Formel (I-A) aufweist, wobei jedes Bio¹, Link¹ und Bio² nach einem der Ansprüche 1 bis 6 definiert ist; oder wobei der Gegenstand eine Mischung aus zwei oder mehr Verbindungen nach Formel (I) und/oder Formel (I-A) umfasst.

10. Gegenstand nach einem der Ansprüche 1 bis 9, wobei die Beschichtung im Wesentlichen frei von jeglichem biologisch aktiven Wirkstoff ist, der verwendet wird, um Bio¹ und/oder Bio² zu bilden und der nicht in einer Verbindung nach Formel (I) oder Formel (I-A) enthalten ist; oder
wobei die Verbindung nach Formel (I) eine reduzierte biologische Aktivität im Vergleich zu dem biologisch aktiven Wirkstoff aufweist, der verwendet wird um Bio¹ und/oder Bio² zu bilden; vorzugsweise, wobei die Verbindung nach Formel (I) oder
Formel (I-A) 0%-20% der biologischen Aktivität des biologisch aktiven Wirkstoff aufweist, der verwendet wird, um Bio¹ und/oder Bio² zu bilden.

11. Gegenstand nach einem der Ansprüche 1 bis 10, wobei die beschichtete Oberfläche ein pharmazeutisch verträgliches Salz der Verbindung nach Formel (I) oder Formel (I-A) umfasst; vorzugsweise, wobei das pharmazeutisch verträgliche Salz das Trifluoracetat- oder Hydrochloridsalz ist.

12. Gegenstand nach einem der Ansprüche 1 bis 11, wobei der Gegenstand eine implantierbare Vorrichtung ist, ausgewählt aus: Prothesenschrittmachern, elektrischen Leitungen, Defibrillatoren, Kunstherzen, ventrikulären Hilfsvorrichtungen,
anatomischen Rekonstruktionsprothesen, künstlichen Herzklappen, Herzklappenstents, Perikard-Patches, chirurgischen Pflastern, Koronarstents, Gefäßtransplantaten, Gefäß- und Strukturstents, Gefäßshunts oder kardiovaskulären Shunts, biologischen Kanälen, Pledgets, Nahtmaterialien, Anuloplastie-Ringen, Stents, Klammern, Herzklappentransplantaten, Hauttransplantaten zur Wundheilung, orthopädischen Spinalimplantaten, orthopädischen Vorrichtungen, Augenimplantaten, Intrauterinvorrichtungen, Stents, maxialen Gesichtsrekonstruktionsauflagen, Zahnimplantaten, intraokularen Linsen, Klemmen, Sternumdrähten, Knochen, Haut, Bändern, Nähten, Herniennetz, Sehnen und Kombinationen daraus.

13. Gegenstand nach einem der Ansprüche 1 bis 11, wobei der Gegenstand eine perkutane Vorrichtung ist, ausgewählt aus der Gruppe bestehend aus: Kathetern, Kanülen, Drainageschläuchen und chirurgischen Instrumenten; vorzugsweise, wobei die perkutane Vorrichtung ein chirurgisches Instrument ist, ausgewählt aus der Gruppe bestehend aus Zangen, Retraktoren, Nadeln und Kathetermanschetten.

14. Gegenstand nach einem der Ansprüche 1 bis 13, wobei die Beschichtung auf der beschichteten Oberfläche eine Dicke zwischen 0,5 und 120 µm aufweist.

15. Verfahren zur Herstellung des Gegenstands nach Anspruch 1, das Verfahren umfassend das In-Kontakt-Bringen der Oberfläche eines Gegenstands mit einer Zusammensetzung, die Zusammensetzung umfassend:
(a) die Verbindung nach einem der Ansprüche 1 bis 8, vorzugsweise, wobei die Konzentration der Verbindung in der Zusammensetzung zwischen 0,05-150 mg/ml liegt, und
(b) ein geeignetes Medium, in dem die Verbindung von (a) löslich ist; vorzugsweise, wobei das geeignete Medium ein organisches Lösungsmittel oder ein wässriges Lösungsmittel ist; vorzugsweise, wobei das geeignete Medium Tetrahydrofuran oder N,N-Dimethylformamid ist.

## Revendications

1. Article comprenant une surface revêtue, où ladite surface revêtue comprend un composé ayant une structure selon la formule (I) :
Bio¹-Link¹-(Bio²-R¹)ₘ (I),
ou un sel pharmaceutiquement acceptable de celui-ci, où
Bio¹ et chaque Bio² sont formés à partir d'un agent biologiquement actif ;
m est 1, 2, 3, 4 ou 5 ;
où chaque Bio² comprend une liaison covalente avec Link¹ ;
R¹ est absent ;
Link¹ est un segment organique, d'organosilicium ou d'organosulfone oligomérique ayant une masse moléculaire entre 60 et 2000 Daltons ;
où ledit article est un dispositif médical implantable ou percutané ; et
où ledit agent biologiquement actif est un agent anti-inflammatoire.

2. Article selon la revendication 1, où ledit composé a une structure selon la formule (I-A):
Bio¹-Link¹-Bio²-R¹ (I-A),
ou un sel pharmaceutiquement acceptable de celui-ci, où
Bio¹ et Bio² sont formés l'un et l'autre à partir dudit agent biologiquement actif ;
R¹ est absent ; et
Link¹ est un segment organique, d'organosilicium ou d'organosulfone oligomérique ayant une masse moléculaire entre 60 et 2000 Daltons.

3. Article selon la revendication 1 ou 2, où ledit agent anti-inflammatoire est choisi dans le groupe consistant en l'amfénac, l'acéclofénac, l'oxacéprol, l'énoxolone, l'hydrocortisone et l'ibuprofène.

4. Article selon l'une quelconque des revendications 1 à 3, où Link¹ est formé à partir d'un diol, d'une diamine ou d'un α,ω-aminoalcool.

5. Article selon l'une quelconque des revendications 1 à 3, où Link¹ est formé à partir d'un poly(oxyde d'éthylène) ayant des groupes amino ou hydroxyle terminaux, et où Link¹ comprend 1-20 unités répétées d'oxyde d'éthylène.

6. Article selon l'une quelconque des revendications 1 à 3, où Link¹ est formé à partir d'un composé choisi dans le groupe consistant en: éthylèneglycol; butanediol; hexanediol; hexaméthylènediol; 1,5-pentanediol; 2,2-diméthyl-1,3-propanediol; 1,4-cyclohexanediol; 1,4-cyclohexanediméthanol; tri(éthylèneglycol); poly(éthylèneglycol), où la masse moléculaire est entre 100 et 2000 Daltons; poly(oxyde d'éthylène)diamine, où la masse moléculaire est entre 100 et 2000 Daltons; esters de lysine; siliconediols; siliconediamines; polyétherdiols; polyétherdiamines; carbonatediols; carbonatediamines; dérivés dihydroxyvinyliques; dihydroxydiphénylsulfone; éthylènediamine; hexaméthylènediamine; 1,2-diamino-2-méthylpropane; 3,3-diamino-n-méthyldipropylamine; 1,4-diaminobutane; 1,7-diaminoheptane; et 1,8-diaminooctane.

7. Article selon l'une quelconque des revendications 1 à 3, où Link¹ est formé à partir de tri(éthylèneglycol).

8. Article selon l'une quelconque des revendications 1 à 3, où Link¹ est formé à partir d'un composé dicarboxylique ou d'un diisocyanate.

9. Article selon l'une quelconque des revendications 1 à 8, où ledit revêtement comprend un second composé ayant une structure selon la formule (I) ou la formule (I-A), où chaque Bio¹, Link¹ et Bio² est comme défini dans l'une quelconque des revendications 1 à 6 ; ou bien
où ledit article comprend un mélange de deux ou plusieurs composés selon la formule (I) et/ou la formule (I-A).

10. Article selon l'une quelconque des revendications 1 à 9, où ledit revêtement est sensiblement dépourvu de tout agent biologiquement actif utilisé pour former Bio¹ et/ou Bio² qui n'est pas inclus dans un composé selon la formule (I) ou la formule (I-A) ; ou bien
où ledit composé selon la formule (I) a une activité biologique réduite comparé à l'agent biologiquement actif utilisé pour former Bio¹ et/ou Bio² ; de préférence, où ledit composé selon la formule (I) ou la formule (I-A) a 0%-20% de l'activité biologique de l'agent biologiquement actif utilisé pour former Bio¹ et/ou Bio².

11. Article selon l'une quelconque des revendications 1 à 10, où ladite surface revêtue comprend un sel pharmaceutiquement acceptable du composé selon la formule (I) ou la formule (I-A); de préférence où ledit sel pharmaceutiquement acceptable est le trifluoroacétate ou le sel chlorhydrate.

12. Article selon l'une quelconque des revendications 1 à 11, où ledit article est un dispositif implantable choisi parmi : les stimulateurs cardiaques prothétiques, les conducteurs électriques, les défibrillateurs, les coeurs artificiels, les dispositifs d'assistance ventriculaire, les prothèses de reconstruction anatomique, les valves cardiaques artificielles, les stents à valves cardiaques, les patchs péricardiques, les patchs chirurgicaux, les stents coronariens, les greffes vasculaires, les stents vasculaires et structuraux, les shunts vasculaires ou cardiovasculaires, les conduits biologiques, les compresses, les sutures, les anneaux d'annuloplastie, les stents, les agrafes, les greffes à valves, les greffes dermiques pour la cicatrisation des plaies, les implants spinaux orthopédiques, les dispositifs orthopédiques, les implants ophtalmiques, les dispositifs intra-utérins, les stents, les placages de reconstruction faciale maxillaire, les implants dentaires, les lentilles intra-oculaires, les clips, les fils sternaux, les os, la peau, les ligaments, les sutures, les treillis pour les hernies, les tendons, et leurs combinaisons.

13. Article selon l'une quelconque des revendications 1 à 11, où ledit article est un dispositif percutané choisi dans le groupe consistant en: les cathéters, les canules, les tubes de drainage, et les instruments chirurgicaux; de préférence, où ledit dispositif percutané est un instrument chirurgical choisi dans le groupe consistant en les forceps, les rétracteurs, les aiguilles et les gaines de cathéter.

14. Article selon l'une quelconque des revendications 1 à 13, où le revêtement sur ladite surface revêtue a une épaisseur entre 0,5 et 120 µm.

15. Procédé pour produire l'article selon la revendication 1, ledit procédé comprenant la mise en contact de la surface d'un article avec une composition, ladite composition comprenant:
(a) le composé selon l'une quelconque des revendications 1 à 8, de préférence, où la concentration dudit composé dans ladite composition est entre 0,05-150 mg/mL, et
(b) un milieu approprié dans lequel le composé de (a) est soluble ; de préférence où ledit milieu approprié est un solvant organique ou un solvant aqueux ; de préférence, où ledit milieu approprié est le tétrahydrofurane ou le *N*,*N-*diméthylformamide.
